**Europäisches Patentamt**

**European Patent Office**

**Office européen des brevets**

(19)

(11) Veröffentlichungsnummer: **0 372 151**
**A1**

(12) # EUROPÄISCHE PATENTANMELDUNG

(21) Anmeldenummer: **89106485.9**

(22) Anmeldetag: **12.04.89**

(51) Int. Cl.5: **A61B 17/22**

Die Bezeichnung der Erfindung wurde geändert (Richtlinien für die Prüfung im EPA, A-III, 7.3).

(30) Priorität: **06.10.88 DE 3833991**

(43) Veröffentlichungstag der Anmeldung:
**13.06.90 Patentblatt 90/24**

(84) Benannte Vertragsstaaten:
**AT BE CH DE ES FR GB GR IT LI LU NL SE**

(71) Anmelder: **Messerschmitt-Bölkow-Blohm Gesellschaft mit beschränkter Haftung Robert-Koch-Strasse D-8012 Ottobrunn(DE)**

(72) Erfinder: **Hessel, Stefan, Dr. Evastrasse 34 D-8000 München 81(DE)**

(54) **Katheter, insbesondere Laserkatheter.**

(57) Bei den Kathetern nach der Hauptanmeldung P 37 17 525, bei dem ein Laserlichtbündel (40) schräg in eine Lichtleitfaser (2) eingekoppelt wird derart, daß es am distalen Katheterende mit zirkumferenzieller Abstrahlcharakteristik in Form eines Kegelmantels seitlich gerichtet austritt, wird erfindungsgemäß im Auskoppelwinkelbereich (46) des zirkumferenziell abgestrahlten Lichtbündels eine Strahlungsabsorptionsfläche (48) angeordnet, über die ein am distalen Katheterende befindliches Applikationsteil (6) aufgeheizt wird, und unbabhängig vom ersten, zirkumferenziellen wird ein zweites, ablativ wirkendes Laserlichtbündel (32) in die proximale Lichtleiter-Stirnfläche (16) eingekoppelt derart, daß es am distalen Lichtleiterende (36) vom ersten Lichtbündel getrennt mit im wesentlichen axialer Abstrahlcharakteristik auf einen zur Katheterspitze verlaufenden Lichtführungskanal (10) gerichtet emittiert und nach außen abgestrahlt wird, wodurch sich zwei verschiedenartige einander ergänzende Laserlichtwirkungen, nämlich der Kauterisierungseffekt des beheizten Applikationsteils einerseits und die direkte Bestrahlungswirkung des nach außen emittierten Lichtbündels andererseits, auf einfache Weise ohne Katheterwechsel und ohne Vergrößerung des Katheterdurchmessers jeweils unabhängig voneinander dosierbar für eine wirksame Gefäßrekanalisation kombinieren lassen.

FIG. 1

# KATHETER

Die Erfindung bezieht sich auf einen Katheter insbesondere zur Behandlung von arteriosklerotisch verengten, organischen Gefäßen nach dem Oberbegriff des Patentanspruchs 1.

Aus der Hauptanmeldung, auf die voll inhaltlich Bezug genommen wird, ist eine als Katheter ausgebildete Einrichtung zur zirkumferenziellen Bestrahlung von durch Ablagerungen verengten Blußgefäßen mit einer die optische Strahlung führenden, flexiblen Lichtleitfaser bekannt, bei welcher die optische Strahlung derart in die Lichtleitfaser eingekoppelt wird, daß sie am distalen Faserende mit seitlicher Richtung in Form eines Kegelmantels über einen an der Katheterspitze angeordneten, transparenten Dilatationskörper austritt, wobei als optische Strahlung eine koagulierend wirkende Laserlichtstrahlung benutzt wird, durch die die Gefäßwand bzw. das an dieser befindliche, stenosierende Plaque im dilatierten Zustand stabilisiert wird. Bei Hohlorganen jedoch, die sehr stark und/oder durch harte, kalzifizierte Ablagerungen verengt sind, reicht die Dilatations- und Koagulationswirkung eines solchen Katheters zur wirksamen Gefäßrekanalisation häufig nicht aus.

Ferner ist aus der US-PS 4 662 368 ein Laserkatheter mit aufheizbarer Metallspitze bekannt, bei dem ein einziges, am distalen Ende eines Lichtleiters mit axialer Abstrahlcharakteristik emittiertes Laserlichtbündel zum einen Teil von der Metallspitze absorbiert wird, die dadurch aufgeheizt wird, und zum anderen Teil über einen in der Metallspitze ausgebildeten, durch ein Saphirfenster geschützten Lichtführungskanal unmittelbar auf die Applikationsstelle abgestrahlt wird. Mit diesem bekannten Katheter läßt sich eine doppelte Ablationswirkung, nämlich einerseits durch die aufgeheizte Metallspitze und andererseits durch die direkte Laserlichtbestrahlung erzielen, und durch entsprechende Steuerung der Laserleistung ist es ohne Katheterwechsel möglich, beide Einzeleffekte gemeinsam verhältnisgleich zu verändern.

Aufgabe der Erfindung ist es, einen Katheter der eingangs genannten Art so auszubilden, daß allein durch proximalseitige Steuereingriffe einerseits der am distalen Katheterende zu Heizzwecken absorbierte und andererseits der nach außen emittierte, direkte Strahlungsanteil unabhängig voneinander jeweils individuell dosierbar sind.

Diese Aufgabe wird erfindungsgemäß durch den im Patentanspruch 1 gekennzeichneten Katheter gelöst.

Erfindungsgemäß werden die beiden erwähnte Einzeleffekte, nämlich das Aufheizen des Applikationsteils einerseits und die Direktbestrahlung der Applikationsstelle andererseits, unabhängig vonein

ander durch zwei verschiedenartige Lichtbündel erzielt, die derart in die Lichtleiteranordnung eingekoppelt werden, daß sie am distalen Lichtleiterende mit jeweils unterschiedlicher Abstrahlcharakteristik getrennt voneinander in räumlich gegenseitig abgegrenzten Auskoppelwinkelbereichen austreten, ohne daß es im Bereich des distalen Katheterendes irgendwelcher Trenn- oder gar Steuerorgane für die emittierten Lichtbündel bedarf. Somit gelingt es auf einfache Weise und allein durch proximalseitige Steuer eingriffe, für die - anders als am distalen Katheterende - in baulicher Hinsicht ausreichend Raum vorhanden ist, die Heizwirkung des zirkumferenziell abgestrahlten Lichtbündels und die direkte Bestrahlungswirkung des axial emittierten Lichtbündels wechselweise oder wahlweise simultan unabhängig voneinander jeweils individuell zu dosieren und daher durch eine bedarfsgerechte Einzeleffektsteuerung eine wirksame, ablative Gefäßrekanalisation zu gewährleisten.

In weiterer vorteilhafter Ausgestaltung der Erfindung ist gemäß Anspruch 2 im Lichtführkanal für das axial abgestrahlte Lichtbündel vorzugsweise ein lichtdurchlässiges Verschlußstück angeordnet, wodurch ein Eindringen von Schmutzstoffen in den Lichtführungskanal und zum Lichtleiterende hin auf einfache Weise verhindert wird. In baulich besonders bevorzugter Ausgestaltung besteht das Verschlußstück gemäß Anspruch 3 aus einem sich im wesentlichen über die gesamte Kanallänge erstrekkenden Lichtleiterstück und ist gemäß Anspruch 4 aus Gründen einer hohen Verschleißfestigkeit vorzugsweise als Saphirlichtleiter ausgebildet. Als Applikationsteil zur Gefäßrekanalisation wird gemäß Anspruch 5 zweckmäßigerweise eine tropfen- oder olivenförmig ausgebildete Metallkappe mit zentrisch verlaufendem Lichtführungskanal verwendet.

Gemäß einem weiteren, besonders bevorzugten Aspekt der Erfindung ist nach Anspruch 6 zur Übertragung beider voneinander unabhängiger Lichtbündel eine einzige, gemeinsame Lichtleitfaser vorgesehen, in die die Lichtbündel auf der Eintrittsseite unter jeweils unterschiedlichen Einkoppelwinkeln zur Flächennormalen der proximalen Lichtleiter-Stirnfläche eingekoppelt werden derart, daß sie aufgrund des spezifischen Transmissionsverhaltens einer Multimode-Lichtleitfaser an der dista len Faser-Stirnfläche getrennt voneinander mit jeweils unterschiedlichen Auskoppelwinkeln jeweils auf die Strahlungsabsorptionsfläche des Applikationsteils bzw. auf den Lichtführungskanal gerichtet austreten. Durch die erfindungsgemäße Mehrfachnutzung der Multimode-Einzelfaser mit selbsttätiger Trennung der unabhängig voneinander steuerbaren Lichtbündel am distalen Lichtfaserende wird eine ganz wesentliche bauliche Vereinfachung mit minimalem Katheterdurchmesser erreicht.

Im Hinblick auf eine günstige räumliche Aufteilung der einzelnen Winkelbereiche innerhalb der numerischen Apertur der Lichtleitfaser besteht der Ein- und zugeordnete Auskoppelwinkelbereich für das axial abgestrahlte Lichtbündel gemäß Anspruch 7 zweckmäßigerweise aus einem zur Faser-Längsachse zentrischen Innenkegel, während der Ein- und zugeordnete Auskoppelwinkelbereich für das zirkumferenziell abgestrahlte Lichtbündel durch einen zum Innenkegel koaxialen Ringkegel gebildet wird, wobei die beiden Lichtbündel gemäß Anspruch 8 vorzugsweise jeweils als innerhalb des zugehörigen Einkoppelwinkelbereichs konvergente, auf die proximale Lichtfaser-Stirnfläche fokussierte Lichtkegel ausgebildet sind.

Zur Erzeugung der beiden Lichtbündel wahlweise simultan oder zeitlich nacheinander und mit jeweils individuell dosierbarer Intensität sind gemäß Anspruch 9 vorzugsweise zwei getrennte Laserlichtquellen mit nachgeschalteter, einkoppelseitiger Optik vorgesehen, oder die beiden Lichtbündel werden in weiterer baulicher Vereinfachung gemäß Anspruch 10 mit jeweils unterschiedlicher Wellenlänge durch eine einzige Laserlichtquelle mit einem nachgeschalteten, die Laserstrahlung wellenlängenabhängig ablenkenden Prisma erzeugt, wobei zur selektiven Steuerung der Lichtbündel gemäß Anspruch 11 vorzugsweise ein umschaltbares optisches Element im Strahlengang der einkoppelseitigen Optik angeordnet ist, welches eine baulich einfache Umschaltung des Katheters vom Heiz- auf den Direktbestrahlungsmodus und umgekehrt ermöglicht. Aus Herstellungsgründen schließlich sind der zur Katheterspitze verlaufende Lichtführungskanal und der das distale Lichtleiterende aufnehmende, von den Lichtbündeln durchsetzte Hohlraum des Applikationsteils gemäß Anspruch 12 vorzugsweise koaxial zueinander ausgebildet.

Die Erfindung wird nunmehr anhand der in den Figuren schematisch dargestellten Ausführungsbeispiele näher beschrieben. Es zeigen

Fig. 1 ein Kathetersystem zur Behandlung eines arteriosklerotisch verengten Blutgefäßes im Direktbestrahlungsmodus (a) und im Heizmodus (b);

Fig. 2 das Kathetersystem gemäß Fig. 1 mit einer modifizierten Laserlichtquelle.

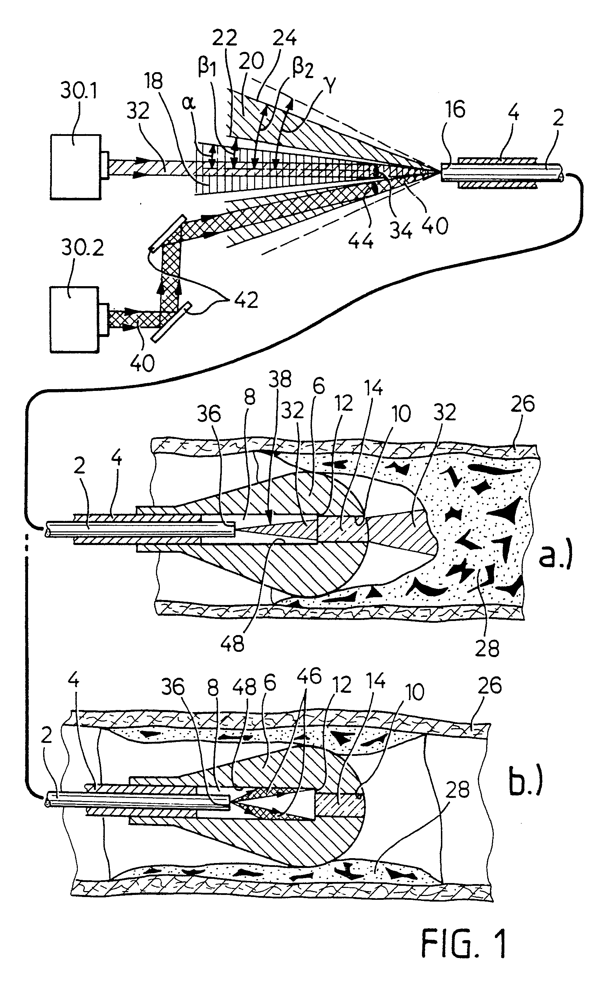

Gemäß Fig. 1 enthält das Kathetersystem eine einzelne flexible Multimode-Lichtleitfaser 2, die in einer flexiblen Faserhülle 4 angeordnet ist, an welcher am distalen Katheterende ein Applikationsteil 6 in Form einer oliven- oder tropfenförmig ausgebildeten Metallkappe befestigt ist. Diese begrenzt einen zentralen, gasgefüllten Hohlraum 8, in welchem das distale Faserende mündet, sowie einen koaxial zum Hohlraum 8 sich zur Katheterspitze erstreckenden Lichtführungskanal 10, der sich an den Hohlraum 8 über eine stufenförmig abgesetze Ringfläche 12 anschließt und durch einen den Lichtführungskanal 10 ausfüllenden Saphir-Lichtleiter 14 flüssigkeitsdicht verschlossen ist.

Auf der senkrecht zur Faser-Längsachse verlaufenden Einkoppel-Stirnfläche 16 sind der Lichtleitfaser 2 innerhalb des in Fig. 1 gestrichelt gezeichneten Akzeptanzkegels (Akzeptanzwinkel $\gamma$) zwei unterschiedliche Einkoppelwinkelbereiche 18 und 20 zugeordnet, von denen der eine - 18 - durch einen zur Faserlängsachse koaxialen Innenkegel mit dem halben Scheitelwinkel $\alpha$ und der zweite - 20 - durch einen zum Innenkegel 18 koaxialen und diesen ringförmig umschließenden Außenkegel gebildet wird, welch lezterer von zwei Kegelmantelflächen 22 und 24, die einen gegenüber dem Winkel $\alpha$ stufenweise erhöhten Öffnungswinkel $\beta_1$ bzw. $\beta_2$ besitzen, begrenzt wird.

Zur Rekanalisierung eines arteriosklerotisch veränderten Blutgefäßes 26 wird der Katheter im Gefäßlumen bis zum stenosierenden Plaque 28 vorgeschoben und ein von einer Laserlichtquelle 30.1 erzeugtes Lichtbündel 32, das eine hauptsächlich ablative, vaporisierende Wirkung auf das Plaque 28, also eine Wellenlänge von z. B. 2,94 $\mu$m besitzt, mittels einer Fokussierlinse 34 innerhalb des Einkoppelwinkelbereichs 18 auf die Lichtfaser-Stirnfläche 16 fokussiert, so daß es an der distalen, ebenfalls senkrecht zur Lichtfaser-Längsachse verlaufenden Lichtfaser-Stirnfläche 36 mit axialer Abstrahlcharakteristik innerhalb eines dem Einkoppelwinkelbereich 18 geometrisch entsprechenden, zentralkegelförmigen Auskoppelwinkelbereichs 38 in den Hohlraum 8 austritt und, da der Auskoppelwinkelbereich 38 vollständig von der inneren Stirnfläche des Saphirstücks 14 überdeckt wird, über dieses an der distalen Katheterspitze ausgekoppelt wird (Fig. 1a).

Aufgrund der direkten, ablativen Bestrahlungswirkung des Lichtbündels 32 wird das in dessen Bestrahlungsbereich liegende Plaque 28 einschließlich der darin vorhandenen, harten, kalzifizierten Ablagerungen vaporisiert, woraufhin der Katheter in das auf diese Weise erweiterte Gefäßlumen vorgeschoben, der Laser 30.1 deaktiviert und ein von einer zweiten Laserlichtquelle 30.2 erzeugtes Lichtbündel 40, das eine hohe Heizwirkung und eine Wellenlänge von z. B. 1,06 $\mu$m besitzt, über ein Spiegelsystem 42 und eine weitere Fokussierlinse 44 in Form eines innerhalb des zweiten Einkoppelwinkelbereichs 20 liegenden Lichtkegels auf die Lichtfaser-Stirnfläche 16 fokussiert wird. Aufgrund der andersartigen Einkoppelung tritt dieses Lichtbündel 40 am distalen Lichtfaserende mit zirkumferenzieller Abstrahlcharakteristik in einem den Einkoppelwinkelbereich 20 bezüglich der Flächennormalen der Lichtfaser-Stirnflächen 16 bzw. 36 geometrisch entsprechenden Auskoppelwinkelbereich 46, also in Form des in Fig. 1b kreuzschraffierten Ringkegels, in den Hohlraum 8 aus, so daß es, durch den stufenförmigen Absatz 12 von der Eintrittsfläche des Saphir-Lichtleiters 14 getrennt, vollständig auf die als Strahlungsabsorptionsfläche ausgebildete zylindrische Wandung 48 der Metallkappe 6 schräg gerichtet auftrifft und somit die Metallkappe 6 aufheizt. Unter der Heizwirkung der Metallkappe 6 werden die angrenzenden, fetthaltigen Ablagerungen kauterisiert und die Gefäßwände im neu geschaffenen Lumen geglättet.

Durch Steuerung der Betriebsparameter, z. B. der Lichtintensität, der Bestrahlungsdauer oder der Betriebsart der beiden Laserlichtquellen 30 können die durch die Lichtbündel 32 und 40 jeweils erzielten Einzeleffekte unabhängig voneinander individuell dosiert werden. Eine wechselweise Umschaltung von dem einen auf das andere Lichtbündel läßt sich nicht nur durch entspre chende Steuerung der beiden Lichtquellen 30, sondern auch durch umschaltbare, optische Elemente erreichen, die - wie das Spiegelsystem 42 - im Strahlengang der Laserlichtbündel angeordnet sind. Die in Fig. 1 gezeigte Anordnung gestattet natürlich auch die simultane Applikation beider Laserlichtbündel 32, 40.

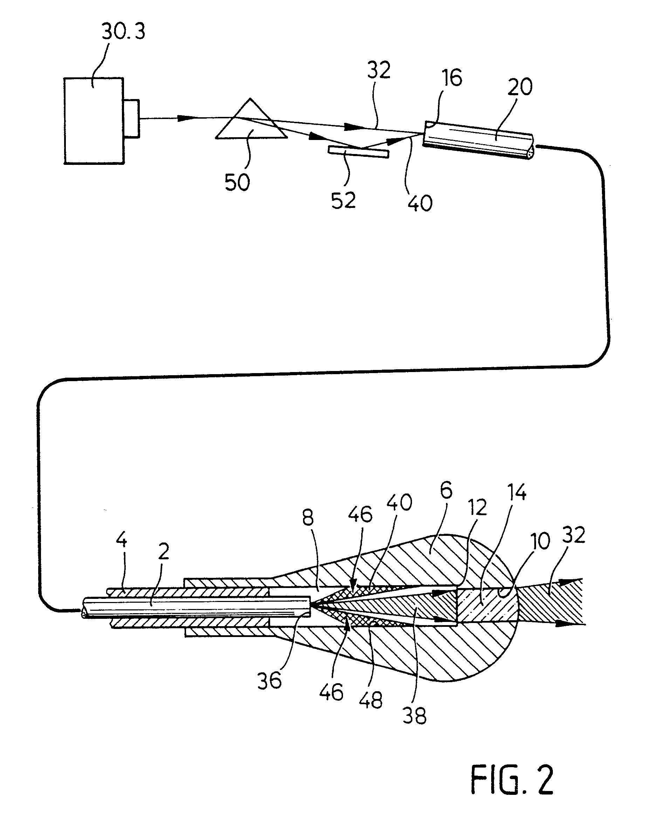

Das in Fig. 2 gezeigte Ausführungsbeispiel unterscheidet sich von dem Kathetersystem nach Fig. 1 im wesentlichen nur durch eine andere Art des einkoppelseitigen Lichterzeugungssystems. Die dem ersten Ausführungsbeispiel entsprechenden Bauelemente sind daher durch das gleiche Bezugszeichen gekennzeichnet. Bei dem Kathetersystem gemäß Fig.2 ist nur eine einzige Lichtquelle 30.3 vorhanden, etwa in Form eines Neodym-YAG-Lasers, der zwischen zwei unterschiedlichen Wellenlängen, nämlich einer direkt wirkenden Laserstrahlung von z. B. 1,32 $\mu$m und einer die Metallkappe 6 aufheizenden Laserstrahlung von z. B. 1,06 $\mu$m, umschaltbar ist. Durch ein dem Laser 30.3 nachgeschaltetes Prisma 50 wird die Laserstrahlung wellenlängenabhängig abgelenkt, so daß das langwelligere Laserlicht in der der Fig. 1 entsprechenden Weise zentral in die Faser-Stirnfläche 16 eingekoppelt wird und an dem distalen Katheterende axial gerichtet über den Saphir-Lichtleiter 14 an der Katheterspitze austritt, während die stärker abgelenkte kurzwelligere Laserstrahlung über einen Spiegel 52 innerhalb des Einkoppelwinkelbereichs 20 gemäß Fig. 1 schräg in die Lichtleitfaser 2 eingekoppelt und am distalen Katheterende in Form eines Kegelmantels seitlich gerichtet abgestrahlt und von der zylindrischen Begrenzungsfläche 48 des Hohlraums 8 wärmeerzeugend absorbiert wird.

Im übrigen ist die Bau- und Betriebsweise des in Fig. 2 gezeigten Kathetersystems die gleiche wie bei dem ersten Ausführungsbeispiel.

**Ansprüche**

1. Katheter, insbesondere zur Behandlung von arteriosklerotisch verengten, organischen Gefäßen mittels optischer Strahlung hoher Intensität, mit einer licht-, insbesondere laserlichtübertragenden, flexiblen Lichtleiteranordnung, in die ein Lichtbündel unter einem vorgegebenen Winkel zur Flächennormalen der proximalen Lichtleiter-Stirnfläche eingekoppelt wird derart, daß es am distalen Lichtleiterende mit zirkumferenzieller Abstrahlcharakteristik in Form eines Kegelmantels seitlich gerichtet austritt, nach der Deutschen Patentanmeldung P 37 17 525,
dadurch **gekennzeichnet**, daß
im Bereich des distalen Katheterendes ein strahlungsabsorbierendes, im Auskoppelwinkelbereich (46) des zirkumferenziell abgestrahlten Lichtbündels (40) liegendes und von diesem beheiztes Applikationsteil (6) und ein außerhalb des Strahlungsbereichs des zirkumferenziellen Lichtbündels sich zwischen distalem Lichtleiterende (36) und Katheterende erstreckender Lichtführungskanal (10) vorgesehen sind und in die Lichtleiteranordnung (2) unabhängig vom zirkumferenziellen, ersten ein zweites Lichtbündel (32) unter einem vorgegebenen zweiten Winkel zur Flächennormalen der proximalen Lichtleiter-Stirnfläche (16) eingekoppelt wird derart, daß es am distalen Lichtleiterende mit im wesentlichen axialer Abstrahlcharakteristik getrennt vom ersten Lichtbündel auf den Lichtführungskanal (10) gerichtet austritt.

2. Katheter nach Anspruch 1,
dadurch **gekennzeichnet**, daß
im Lichtführungskanal (10) für das zweite Lichtbündel (32) ein lichtdurchlässiges Verschlußstück (14) angeordnet ist.

3. Katheter nach Anspruch 2,
dadurch **gekennzeichnet**, daß
das Verschlußstück (14) aus einem sich im wesentlichen über die gesamte Kanallänge erstreckenden Lichtleiterstück besteht.

4. Katheter nach Anspruch 2 oder 3,
dadurch **gekennzeichnet**, daß
das Verschlußstück (14) ein Saphir-Lichtleiterstück ist.

5. Katheter nach einem der vorhergehenden Ansprüche,
dadurch **gekennzeichnet**, daß
als Applikationsteil (6) eine tropfenförmig ausgebildete Metallkappe mit zentrisch verlaufendem Lichtführungskanal (10) vorgesehen ist.

6. Katheter nach ein der vorhergehenden Ansprüche,
dadurch **gekennzeichnet**, daß
zur Übertragung beider Lichtbündel (32, 40) eine einzige gemeinsame Lichtleitfaser (2) vorgesehen ist und dieser auf der Eintrittsseite zwei unterschiedliche Einkoppelwinkelbereiche (18, 20) und auf der Austrittsseite zwei den Einkoppelwinkelbereichen geometrisch entsprechende, unterschiedliche, jeweils auf die Strahlungsabsorptionsfläche (48) des Applikationsteils (6) bzw. auf den Lichtführungskanal (10) gerichtete Auskoppelwinkelbereiche (46, 38) zugeordnet sind und jeder Ein- und zugehörige Auskoppelwinkelbereich der Einzelfaser von jeweils einem der voneinander unabhängigen Lichtbündel (32, 40) durchsetzt ist.

7. Katheter nach Anspruch 6,
dadurch **gekennzeichnet**, daß
der Ein- und zugehörige Auskoppelwinkelbereich (18, 38) für das zweite Lichtbündel (32) durch einen zur Faser-Längsachse zentrischen Innenkegel und der Ein- und zugehörige Auskoppelwinkelbereich (20, 46) für das erste Lichtbündel (40) durch einen zum Innenkegel koaxialen Ringkegel gebildet ist.

8. Katheter nach Anspruch 6 oder 7,
dadurch **gekennzeichnet**, daß
die Lichtbündel (32, 40) jeweils als innerhalb des zugeordneten Einkoppelwinkelbereichs (18 bzw. 20) konvergenter, auf die proximale Lichtleitfaser-Stirnfläche (16) fokussierter Lichtkegel ausgebildet sind.

9. Katheter nach einem der vorhergehenden Ansprüche,
dadurch **gekennzeichnet**, daß
zwei getrennte Laserlichtquellen (30.1 und 30.2) zur Erzeugung der beiden Lichtbündel (32,40) und eine einkoppelseitige Optik (34, 42, 44) zur Einkopplung der Lichtbündel unter dem jeweils vorgegebenen Einkoppelwinkel vorgesehen sind.

10. Katheter nach einem der Ansprüche 1 bis 8,
dadurch **gekennzeichnet**, daß
eine einzige Laserlichtquelle (30.3) zur Erzeugung der beiden Lichtbündel (32, 40) mit jeweils unterschiedlicher Wellenlänge und eine einkoppelseitige Optik (50, 52) einschließlich eines die Laserstrahlung wellenlängenabhängig ablenkenden Prismas (50) vorgesehen sind.

11. Katheter nach Anspruch 9 oder 10,
dadurch **gekennzeichnet**, daß
im Strahlengang der einkoppelseitigen Optik (34, 42, 44; 50, 52) mindestens ein umschaltbares optisches Element zur selektiven Steuerung zumindest eines der Lichtbündel (32 oder 40) angeordnet ist.

12. Katheter nach einem der vorhergehenden Ansprüche,
dadurch **gekennzeichnet**, daß
der Lichtführungskanal (10) koaxial von einem im Applikationsteil (6) ausgebildeten, das distale Lichtleiterende (36) aufnehmenden, zentralen Hohlraum (8) zur Katheterspitze verläuft.

FIG. 1

FIG. 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 236 867 (RICHARD WOLF) * Spalte 3, Zeile 33 - Spalte 4, Zeile 4; Figuren 1,2 * --- | 1 | A 61 B 17/22 |
| A,D | WO-A-8 404 879 (LASERSCOPE) * Seite 7, Zeile 29 - Seite 8, Zeile 11; Figuren 1,2 * --- | 1 | |
| A | EP-A-0 195 375 (M.I.T.) * Seite 18, Zeile 18 - Seite 19, Zeile 4; Figur 1 * ----- | 1 | |

RECHERCHIERTE SACHGEBIETE (Int. Cl.5)

A 61 B
A 61 N
B 23 K

Der vorliegende Recherchenbericht wurde für alle Patentansprüche erstellt

| Recherchenort | Abschlußdatum der Recherche | Prüfer |
|---|---|---|
| DEN HAAG | 10-01-1990 | MOERS R.J. |